# EUROPEAN PATENT SPECIFICATION

(11) **EP 1 727 512 B1**
(45) Date of publication and mention of the grant of the patent: **09.11.2011**
(21) Application number: 04735693.6
(22) Date of filing: 01.06.2004
(51) Int. Cl.: A61K 8/04, A61K 8/34, A61K 8/63, A61Q 17/04, A61Q 19/00

(54) **COMPOSITION CONTAINING GINSENOSIDE F1 AND EGCG FOR PREVENTING SKIN DAMAGE**
ZUSAMMENSETZUNG MIT GINSENOSID F1 UND EGCG ZUR PRÄVENTION VON HAUTSCHÄDEN
COMPOSITION DERMO-PROTECTRICE CONTENANT DU GINSENOSIDE F1 ET EGCG

(30) Priority: 26.03.2004 KR 2004020800
(43) Date of publication of application: 06.12.2006
(73) Proprietor: Amorepacific Corporation, Seoul 140-777 (KR)
(72) Inventor: CHO, Si Young, Yongin-si, Kyunggi-do 449-950 (KR); KANG, Byung-Young, Seocho-gu, Seoul 137-769 (KR); YEOM, Myeong Hoon, No. 101-902 Byeoksan Ap., Yongin-si, Kyunggi-do 449-548 (KR); LEE, Tae Ryong, No. 932-1303 Taeyeong Apartment, Suwon-si, Kyunggi-do 442-470 (KR); CHANG, Ih-Seop, No. 102-1104 Dongbo Apartment, Yongin-si, Kyunggi-do 449-846 (KR)
(74) Representative: Poulin, Gérard
(86) International application number: PCT/KR2004/001303
(87) International publication number: WO 2005/092280

(56) References cited:
- EP-A- 1 327 434
- WO-A-03/051287
- KR-A- 2003 064 986
- KR-A- 2004 009 983
- US-A1- 2003 162 725
- US-A1- 2003 175 234
- US-B1- 6 277 396
- US-B1- 6 437 004
- LEE E H ET AL: "Ginsenoside F1 protects human HaCaT keratinocytes from ultraviolet-B-induced apoptosis by maintaining constant levels of Bcl-2" JOURNAL OF INVESTIGATIVE DERMATOLOGY, NATURE PUBLISHING GROUP, GB, vol. 121, no. 3, 1 September 2003 (2003-09-01), pages 607-613, XP008102382 ISSN: 0022-202X [retrieved on 2003-08-18]

## Description

### BACKGROUND OF THE INVENTION

### 1. Field of the Invention

The present invention relates to a composition for preventing skin damage, containing ginsenoside F1 (20-O-β-D-glucopyranosyl-20(S)-protopanaxatriol) and EGCG ((-)epigallocatechin-3-gallate). More particularly, the present invention relates to a composition containing ginsenoside F1 and EGCG at low concentrations, capable of preventing UV-induced skin damage by the synergistic interaction thereof, and to a method for preventing skin damage.

### 2. Description of Prior Art

Human skin, as a primary protective barrier, protects the vital organs of the body from external irritants such as changes in temperature or humidity, ultraviolet rays, and contaminants, and plays an important role in the regulation of biological homeostasis such as thermoregulation. However, the skin is exposed to external surrounding and thereby easily damaged by external irritants. Among these external irritants, ultraviolet rays are the main irritant to cause skin aging and apoptotic cell death in the epidermis.

The most serious cause of skin aging is ultraviolet rays with wavelengths of 280~320nm (UVB). If the skin is exposed to the ultraviolet rays, cellular components such as DNA or proteins may be damaged to form sunburn cells, which activate an enzyme, caspase, and undergo apoptotic pathways accompanied with DNA fragmentation by the action of the enzyme, finally ending in cell death. In this way, apoptosis induces the death of damaged cells, which prevents them from developing into a tumor.

Bcl-2 is a gene that plays an important role in this apoptotic pathway, and encodes 26kDa protein localized at the nuclear membrane and the outer mitochondrial membrane. Bcl-2 protein attaches to a protein favouring the induction of apoptosis such as Bax and hinders the functions thereof, to inhibit the induction of apoptosis. Therefore, susceptibility to undergoing apoptosis may be dependent on the ratio between Bcl-2 protein and Bax protein.

It has been reported that the expression of Bcl-2 is rapidly down-regulated by ultraviolet irradiation in the epidermis. Further, UV-induced apoptosis was inhibited in cells overexpressing Bcl-2 protein. However, the overexpression of Bcl-2 may also inhibit apoptosis in cells having severe DNA damage, which thus may develop a cancer. Therefore, it is very important to specifically regulate the expression of Bcl-2.

EGCG, a major polyphenol in green tea, has a strong antioxidant activity and an activity of scavenging harmful radicals. It is also known that this compound can inhibit inflammatory reactions and skin cancer formation induced by UV irradiation. Recently, EGCG was found to enhance Bcl-2 expression and reduce Bax expression in normal human epidermal keratinocytes, inhibiting UV-induced apoptosis. The EGCG application to squamous carcinoma cells reduced the phosphorylation of Bax protein and thereby inhibited the proliferation of the carcinoma cells. However, the target of EGCG in the signal transduction pathway involved with apoptosis and cell proliferation is not yet known.

Retinoblastoma (Rb) protein is expressed by tumor suppressor and plays an important role in generation, cancer, cell growth and differentiation, and cell death. Rb is a nuclear protein and its phosphorylation is regulated by cell cycle or DNA-damaging factors. Thus, it is involved in the induction of apoptosis together with E2F transcription factors. Further, it is known that the dephosphorylation of Rb protein is inhibited by Bcl-2 overexpression and thereby Rb is stabilized. That is, it is also involved in the inhibition of apoptosis.

However, there has not been any report on a substance capable of regulating apoptosis in human epidermal cells by protecting cells damaged weakly from apoptosis and by inducing apoptosis in cells damaged severely, and thereby capable of protecting the skin, and harmless to human beings in order to be easily applied.

The use of ginenoside F1 or EGCG for preventing skin damage is already known, e.g. from
LEE E H ET AL: "Ginsenoside F1 protects human HaCaT keratinocytes from ultraviolet-B-induced apoptosis by maintaining constant levels of Bcl-2", JOURNAL OF INVESTIGATIVE DERMATOLOGY, NATURE PUBLISHING GROUP, GB, vol. 121, no. 3, 1 September 2003 (2003-09-01), pages 607-613, ISSN: 0022-202X [retrieved on 2003-08-18] and
WO 03051287 A, respectively.

### SUMMARY OF THE INVENTION

Under these circumstances, the present inventors found that a composition containing ginsenoside F1 (20-O-β-D-glucopyranosyl-20(S)-protopanaxatriol) and EGCG ((-)epigallocatechin-3-gallate) having an excellent effect in protecting epidermal cells and preventing the damage thereof, and thereby completed the present invention.

In detail, compositions containing one of ginsenoside F1 and EGCG at a low concentration did not show any skin-protecting effect. However, a composition containing a mixture of ginsenoside F1 and EGCG at the said concentrations showed an excellent skin-protecting effect by the synergistic interaction thereof. That is, a combined treatment with these two compounds can regulate the expression of Bcl-2 against UV irradiation by the synergistic interaction thereof even at low concentrations, where any single treatment shows no effect.

Therefore, the present invention relates to a composition for preventing 5 skin damage, containing ginsenoside F1 represented by the following chemical formula 1 and EGCG represented by the following chemical formula 2 as active ingredients:

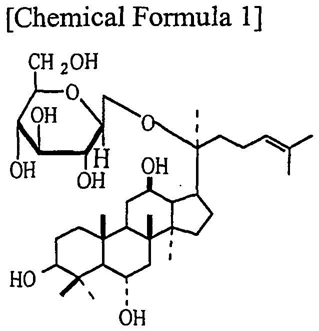

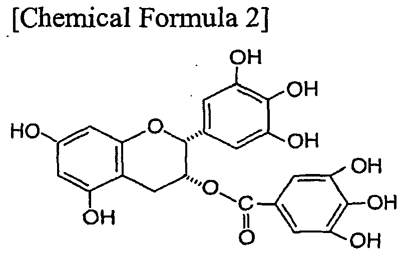

The composition provided by the present invention may be involved in apoptosis of epidermal cells to prevent skin aging and to maintain the function of epidermal cells.

The said ginsenoside F1 and EGCG may be preferably incorporated in a combined amount of 0.0001% to 10% by weight based on the total weight of the composition. Further, the said ginsenoside F1 and EGCG may be preferably incorporated in a weight ratio of 1:0.1~10.

In more detail, the present invention provides an inhibitor of apoptosis induced by a low dose of UV irradiation, containing a combination of ginsenoside F1 and EGCG as an active ingredient.

Further, the present invention provides a regulator of Bcl-2 expression down-regulated by UV irradiation, containing a combination of ginsenoside F1 and EGCG as an active ingredient.

In addition, the present invention provides a regulator of Brn-3a expression down-regulated by UV irradiation, containing a combination of ginsenoside F1 and EGCG as an active ingredient.

### DETAILED DESCRIPTION OF THE INVENTION

According to the present invention, ginsenoside F1 itself cannot enhance the expression of Bcl-2, but can prevent Bcl-2 expression from being down-regulated by UV irradiation. That is, ginsenoside F1 maintains the level of Bcl-2 expression to the normal level by regulating the expression of Brn-3a, a Bcl-2-specific transcription factor, and thereby can inhibit apoptosis in epidermal cells caused by a low dose of UV irradiation. Meanwhile, because a high dose of UV irradiation down-regulates the expression of Bcl-2, and apoptosis in damaged cells may be induced as it is, there is no risk that damaged cells may develop to a skin cancer.

The present invention confirmed the synergistic interaction of ginsenoside F1 with EGCG in the said effect in the examples described later. In detail, a combined treatment with these two compounds at low concentrations, where any single treatment showed no significant effect, recovered Bcl-2 expression down-regulated by UV irradiation to the normal level in human epidermal keratinocyte, HaCaT cells, and thereby inhibited apoptotic cell death. Further, we confirmed, on the basis of the prior researches, that an apoptosis-inhibiting effect in the synergistic interaction with EGCG was obtained by regulating the expression of Brn-3a, a Bcl-2-specific transcription factor. Only the combined treatment with the said two compounds prevented the dephosphorylation of a tumor-suppressing Rb protein and thereby inhibited apoptotic cell death.

The above results indicate that a combined treatment with ginsenoside F1 and EGCG can maintain constant levels of Bcl-2 within cells by the synergistic interaction of these two compounds even at low concentrations, and can prevent the dephosphorylation of Rb protein to inhibit apoptotic cell death.

Therefore, ginsenoside F1 and EGCG can inhibit apoptotic cell death by maintaining constant levels of Bcl-2 within cells by the synergistic interaction thereof. Furthermore, because ginsenoside F1 itself cannot enhance the expression of Bcl-2 and does not protect cells having a possibility to develop a cancer from apoptosis, there is no risk that damaged cells may develop to a cancer. These results suggest the possibility of using a combination of ginsenoside F1 and EGCG as an anti-aging agent in human skin by inhibiting UV-induced apoptosis and preventing cellular damages.

In conclusion, the present invention provides a skin-care composition containing a combination of ginsenoside F1 and EGCG as an active ingredient. The composition of the present invention can prevent UV-caused skin damage and thereby skin aging. The composition may be formulated into skin-care external compositions, for example a cosmetic composition. However, the purpose and the formulation of the said composition may not be limited hereto.

Ginsenoside F1 to be used in the present invention is a compound obtained by dissolving a purified ginseng saponin in an aqueous solvent such as distilled water or buffer solution, or in a mixture of the said aqueous solvent and an organic solvent, and then reacting with at least one of naringinase separated from *Penicillium* and pectinase separated from *Aspergillus.* However, a method for preparing ginsenoside F1 may not be limited hereto.

### BRIEF DESCRIPTION OF THE DRAWINGS

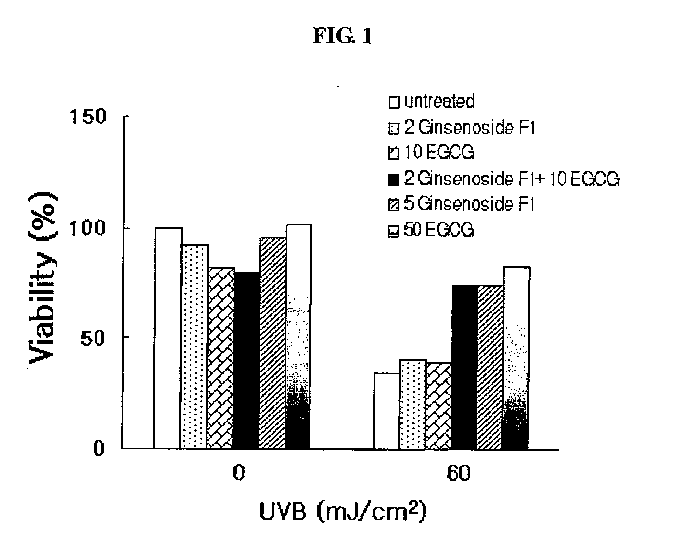

Figure 1 shows graphically the results of MTT-reduction assay for showing the antiapoptotic effect of combined treatment with ginsenoside F1 and EGCG in epidermal cells.

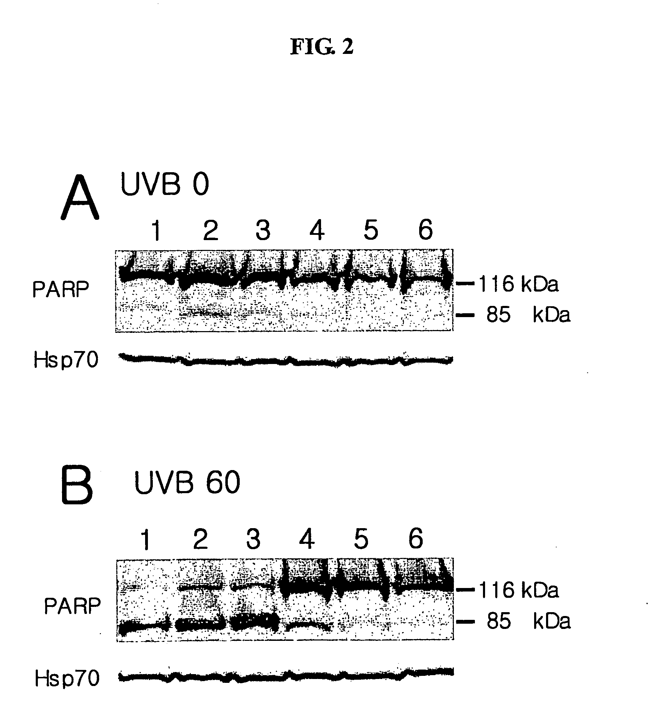

Figure 2 shows the results of Western-blot analysis for showing the inhibitory effect of combined treatment with ginsenoside F1 and EGCG on the cleavage of poly (ADP-ribose) polymerase (PARP). A shows the results in the absence of UV irradiation and B shows the results in the presence of 60 mJ/cm² of UV irradiation. Hsp70 represents that equal amounts of proteins were used. In Figure 2, line 1 shows the result of the untreated control group; line 2 shows the result of the test group treated with 2 µM ginsenoside F1; line 3 treated with 10 µM EGCG; line 4 treated with 2 µM ginsenoside F1 and 10 µM EGCG; line 5 treated with 5 µM ginsenoside F1; and line 6 treated with 50 µM EGCG.

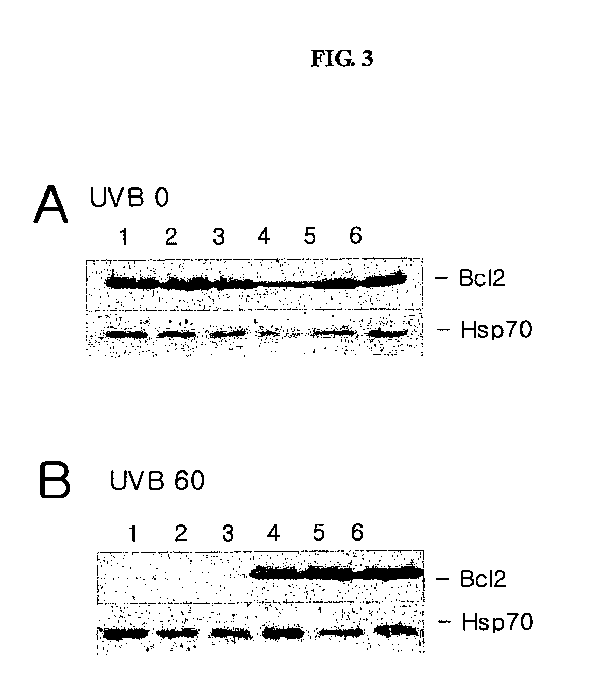

Figure 3 shows the results of Western-blot analysis for showing the inhibitory effect of combined treatment with ginsenoside F1 and EGCG on the down-regulation of Bcl-2 expression. A shows the results in the absence of UV irradiation and B shows the results in the presence of 60 mJ/cm² of UV irradiation. Hsp70 represents that equal amounts of proteins were used. The amount of each test sample treated was equal to that shown in Figure 2.

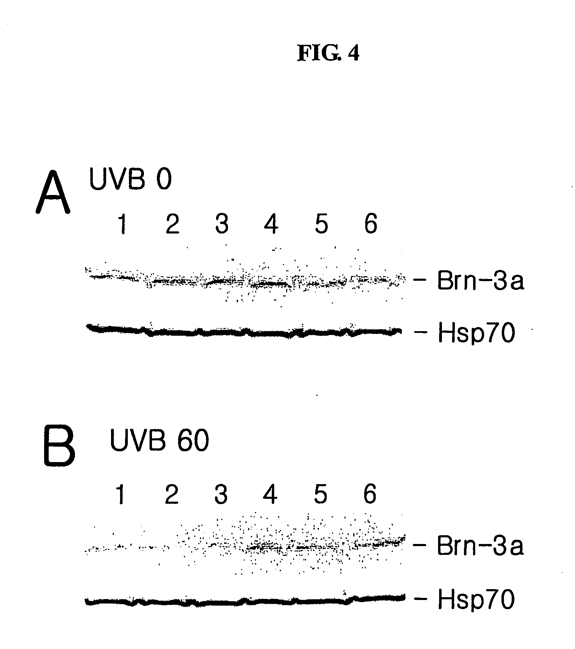

Figure 4 shows the results of Western-blot analysis for showing the inhibitory effect of combined treatment with ginsenoside F1 and EGCG on the down-regulation of Brn-3a expression. A shows the results in the absence of UV irradiation and B shows the results in the presence of 60 mJ/cm² of UV irradiation. Hsp70 represents that equal amounts of proteins were used. The amount of each test sample treated was equal to that shown in Figure 2.

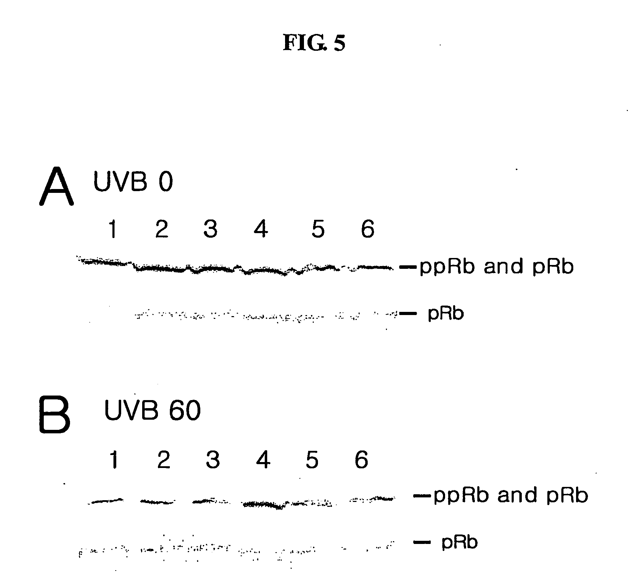

Figure 5 shows the results of Western-blot analysis for showing the inhibitory effect of combined treatment with ginsenoside F1 and EGCG on the dephosphorylation of Rb protein. The upper blots were obtained by using an antibody recognizing all phosphorylated forms of Rb protein and the lower blots were obtained by blotting the same membrane with an antibody recognizing only underphosphorylated Rb protein. A shows the results in the absence of UV irradiation and B shows the results in the presence of 60 mJ/cm² of UV irradiation. The amount of each test sample treated was equal to that shown in Figure 2.

### PREFERRED EMBODIMENT OF THE INVENTION

The present invention will be described in more detail by way of the following examples. However, these examples are provided for the purpose of illustration only and should not be construed as limiting the scope of the invention, which will be apparent to one skilled in the art.

### <Reference Example 1> Preparation of purified ginseng saponin

4 ℓ of methanol containing distilled water were added to 2 kg of red ginseng (KT&G, six-year-old red ginseng) and refluxed three (3) times, then settled down at 15°C for 6 days. The crude extracts were passed through filter paper and centrifuged to separate residues and filtrates. The filtrates were concentrated under reduced pressure. The concentration was suspended in distilled water, and then extracted five (5) times with 1 ℓ of ether to remove pigments. The aqueous part was extracted three (3) times with 500 mℓ of 1-butanol. The obtained 1-butanol parts were treated with 5% KOH and washed with distilled water, then concentrated under reduced pressure. The obtained 1-butanol extract was dissolved in a small amount of methanol and added into a large amount of ethyl acetate. The resulting precipitate was dried, to give 70g of purified ginseng saponin.

### <Reference Example 2> Preparation of ginsenoside F1

10 g of the purified ginseng saponin obtained in Reference Example 1 was dissolved in 1000 mℓ of citrate buffer (pH4.0). 15 g of naringinase separated from *Penicillium* (Sigma, St. Louis, MO) was then added thereto and reacted for 48 hours under stirring in a water bath at 40°C. After the reaction was terminated, the enzyme was deactivated by heating for 10 minutes and the reaction mixture was extracted three (3) times with an equal amount of ethyl acetate, then concentrated. The obtained product was fractionated by silica gel column chromatography using chloroform : methanol (9:1), to give 1.5g of ginsenoside F1.

### <Example 1> Antiapoptotic effect of combined treatment with ginsenoside F1 and EGCG in HaCaT cells :

### [Step 1] Cell line and cell culture

Human keratinocyte HaCaT cell line was provided by Dr. N.E. Fusenig (Deutsches Krebsforschungszentrum(DKFZ), Heidelberg, Germany) and cultured in DMEM (Dulbecco's modified Eagle's medium, Gibco 1210-0038) supplemented with 10% fetal bovine serum. Cultures were incubated at 37°C, in humidified air with 5% CO₂.

### [Step 2] Inhibition of UV-induced apoptosis in HaCaT cells by a combined treatment with ginsenoside F1 and EGCG

Cell lines cultured in Step 1 were treated with trypsin to give a single-cell suspension and seeded into a 6-well microplate at 2×10⁵ cells per well, then cultured for 24 hours. Subsequently, the culture medium was refreshed with serum-free DMEM and cells were cultured for another 24 hours. The microplate was then treated with 2 µM ginsenoside F1; 10 µM EGCG; a combination of 2 µM ginsenoside F1 and 10 µM EGCG; 5 µM ginsenoside F1; and 50 µM EGCG in separate wells.

For reference, ginsenoside F1 was dissolved in 100% ethanol at a 1/1000-fold concentration to the medium and EGCG (Sigma) was dissolved in dimethyl sulfoxide (DMSO) at a 1/1000-fold concentration to the medium, and then added at the required amount to the culture mediums.

After 24-hour treatment of each test sample, each microplate was washed with phosphate buffered saline (PBS) and exposed to 60 mJ/cm² of UVB in the presence of PBS. PBS was then removed and the culture medium was refreshed with a medium containing each compound at the corresponding concentration. As a control, untreated cells were cultured in the same way.

24 hours after UV irradiation, to the microplates treated with each compound or untreated was added 3-(4,5-dimethyl thiazol-2-yl)-2,5-diphenyltetrazolium bromide (MTT, Sigma) solution, and then the cells were cultured at 37°C for 4 hours. DMSO was added and dissolved completely. The optical density (OD) of formazan formed at 540 nm was measured using an ELISA reader (Thermo Max, Molecular Devices Co.). Cell viability in each test group was evaluated as a relative value, considering OD of untreated control cells as 100%. The results are shown in Figure 1.

As shown in Figure 1, single treatments with 2 µM ginsenoside F1 or 10 µM EGCG alone showed no difference in UV-caused apoptotic cell population compared with untreated control cells. However, combined treatment with 2 µM ginsenoside F1 and 10 µM EGCG inhibited UV-caused apoptotic cell death by about 2-fold compared with untreated control cells. This was similar to the antiapoptotic effects obtained by single treatments with either 5 µM ginsenoside F1 or 50 µM EGCG alone.

### <Example 2> Inhibitory effect of combined treatment with ginsenoside F1 and EGCG on the cleavage of PARP protein :

### [Step 1] Cell line and cell culture

Cell lines used in this experiment and the culture thereof were the same as used in Step 1 of Example 1.

### [Step 2] Inhibition of UV-induced PARP cleavage by a combined treatment with ginsenoside F1 and EGCG

Cell lines cultured in Step 1 were treated with trypsin to give a single-cell suspension and seeded into 6-well microplates at 2×10⁵ cells per well, then cultured for 24 hours. Subsequently, the culture medium was refreshed with serum-free DMEM and cells were cultured for another 24 hours. The microplates were then treated with 2 µM ginsenoside F1; 10 µM EGCG; a combination of 2 µM ginsenoside F1 and 10 µM EGCG; 5 µM ginsenoside F1; and 50 µM EGCG, respectively. Ginsenoside F1 was dissolved in 100% ethanol at a 1/1000-fold concentration to the medium and EGCG (Sigma) was dissolved in DMSO at a 1/1000-fold concentration to the medium. After 24-hour treatment of each test sample, each microplate was washed with PBS and exposed to 60 mJ/cm² of UVB in the presence of PBS. PBS was then removed and the culture medium was refreshed with a medium containing each compound at the corresponding concentration. As a control, untreated cells were cultured in the same way.

24 hours after UV irradiation, cells treated with each compound or untreated were washed with PBS and harvested by treating with trypsin. Cells were then lysed in 500 µℓ of protein extraction buffer containing 8M urea, 2% CHAPS, 50 mM DTT, 2M thiourea, 2mM PMSF and 100 µg/µℓ leupeptine at room temperature for 10 minutes. Supernatants were collected by centrifugation at 15,000 x g for 10 minutes at 4°C and protein concentrations were determined using BIO-Rad Protein Dye Reagent^{™}. 20 µg of proteins were separated in size order by 8% SDS-PAGE and transferred to PDF membrane (BioRad) at 50 V for 12 hours. The blots were blocked in a 5% non-fat milk-solution for 1 hour and then reacted with an anti-PARP rabbit polyclonal antibody (Santa Cruz) as a primary antibody and a horse radish peroxidase (HRP)-conjugated anti-rabbit IgG (Amersham) as a secondary antibody using an enhanced chemiluminescence (ECL) kit (Amersham). The blots were then exposed to X-ray film (Fuji) and developed to determine the level of protein expression. The bands on the film were scanned using PowerLook 2100 XL (UMAX) and analyzed with ImageMaster 2D Elite software (Amersham Biosciences). The content of PARP cleaved was evaluated as a relative value on the basis of the content in the untreated control group. The results are shown in Figure 2.

As shown in Figure 2, combined treatment with 2µM ginsenoside F1 and 10µM EGCG inhibited UV-caused cleavage of PARP protein by about 1.4-fold compared with untreated control cells, or with single treatments with an equal concentration of each compound.

### <Example 3> Inhibitory effect of combined treatment with ginsenoside F1 and EGCG on the down-regulation of Bcl-2 expression :

### [Step 1] Cell line and cell culture

Cell lines used in this experiment and the culture thereof were the same as used in Step 1 of Example 1.

### [Step 2] Inhibition of UV-induced down-regulation of Bcl-2 expression by a combined treatment with ginsenoside F1 and EGCG

Cell lines cultured in Step 1 were treated with trypsin to give a single-cell suspension and seeded into 6-well microplates at 2×10⁵ cells per well, then cultured for 24 hours. Subsequently, the culture medium was refreshed with serum-free DMEM and cells were cultured for another 24 hours. The microplates were then treated with 2 µM ginsenoside F1; 10 µM EGCG; a combination of 2 µM ginsenoside F1 and 10 µM EGCG; 5 µM ginsenoside F1; and 50 µM EGCG, respectively. Ginsenoside F1 was dissolved in 100% ethanol at a 1/1000-fold concentration to the medium and EGCG (Sigma) was dissolved in DMSO at a 1/1000-fold concentration to the medium. After 24-hour treatment of each test sample, each microplate was washed with PBS and exposed to 60 mJ/cm² of UVB in the presence of PBS. PBS was then removed and the culture medium was refreshed with a medium containing each compound at the corresponding concentration. As a control, untreated cells were cultured in the same way.

24 hours after UV irradiation, cells treated with each compound or untreated were washed with PBS and harvested by treating with trypsin. Cells were then lysed in 500µℓ of protein extraction buffer containing 8M urea, 2% CHAPS, 50mM DTT, 2M thiourea, 2mM PMSF and 100µg/µℓ leupeptine at room temperature for 10 minutes. Supernatants were collected by centrifugation at 15,000×g for 10 minutes at 4°C and protein concentrations were determined using BIO-Rad Protein Dye Reagent^{™}. 20µg of proteins were separated in size order by 8% SDS-PAGE and transferred to PDF membrane (BioRad) at 50 V for 12 hours. The blots were blocked in a 5% non-fat milk-solution for 1 hour and then reacted with an anti-Bcl-2 rabbit polyclonal antibody (Santa Cruz) as a primary antibody and an HRP-conjugated anti-rabbit IgG (Amersham) as a secondary antibody using an ECL kit (Amersham). The blots were then exposed to X-ray film (Fuji) and developed to determine the level of protein expression. The bands on the film were scanned using PowerLook 2100 XL (UMAX) and analyzed with ImageMaster 2D Elite software (Amersham Biosciences). The results are shown in Figure 3.

As shown in Figure 3, Bcl-2 protein in untreated cells was not expressed by UV irradiation. Further, single treatments with 2 µM ginsenoside F1 or 10 µM EGCG alone showed no difference in Bcl-2 expression compared with the untreated control group. However, combined treatment with 2 µM ginsenoside F1 and 10 µM EGCG maintained the level of Bcl-2 expression in the presence of UV irradiation similar to the level in the absence of UV irradiation. That is, combined treatment with two compounds increased the expression of Bcl-2 by about 3-fold compared with the untreated control group or single treatments with each compound.

### <Example 4> Inhibitory effect of combined treatment with ginsenoside F1 and EGCG on the down-regulation of Brn-3a expression :

### [Step 1] Cell line and cell culture

Cell lines used in this experiment and the culture thereof were the same as used in Step 1 of Example 1.

### [Step 2] Inhibition of UV-induced down-regulation of Brn-3a expression by a combined treatment with ginsenoside F1 and EGCG

Cell lines cultured in Step 1 were treated with trypsin to give a single-cell suspension and seeded into 6-well microplates at 2×10⁵ cells per well, then cultured for 24 hours. Subsequently, the culture medium was refreshed with serum-free DMEM and cells were cultured for another 24 hours. The microplates were then treated with 2 µM ginsenoside F1; 10 µM EGCG; a combination of 2 µM ginsenoside F1 and 10 µM EGCG; 5 µM ginsenoside F1; and 50 µM EGCG, respectively. Ginsenoside F1 was dissolved in 100% ethanol at a 1/1000-fold concentration to the medium and EGCG (Sigma) was dissolved in DMSO at a 1/1000-fold concentration to the medium. After 24-hour treatment of each test sample, each microplate was washed with PBS and exposed to 60 m J /cm² of UVB in the presence of PBS. PBS was then removed and the culture medium was refreshed with a medium containing each compound at the corresponding concentration. As a control, untreated cells were cultured in the same way.

24 hours after UV irradiation, cells treated with each compound or untreated were washed with PBS and harvested by treating with trypsin. Cells were then lysed in 500µℓ of protein extraction buffer containing 8M urea, 2% CHAPS, 50mM DTT, 2M thiourea, 2mM PMSF and 100µg/µℓ leupeptine at room temperature for 10 minutes. Supernatants were collected by centrifugation at 15,000×g for 10 minutes at 4°C and protein concentrations were determined using BIO-Rad Protein Dye Reagent^{™}. 20µg of proteins were separated in size order by 8% SDS-PAGE and transferred to PDF membrane (BioRad) at 50 V for 12 hours. The blots were blocked in a 5% non-fat milk-solution for 1 hour and then reacted with an anti-Brn-3a rabbit polyclonal antibody (Santa Cruz) as a primary antibody and an HRP-conjugated anti-rabbit IgG (Amersham) as a secondary antibody using an ECL kit (Amersham). The blots were then exposed to X-ray film (Fuji) and developed to determine the level of protein expression. The bands on the film were scanned using PowerLook 2100 XL (UMAX) and analyzed with ImageMaster 2D Elite software (Amersham Biosciences). The results are shown in Figure 4.

As shown in Figure 4, the expression of Brn-3a protein was decreased by UV irradiation and recovered only by combined treatment with two compounds.

### <Example 5> Inhibitory effect of combined treatment with ginsenoside F1 and EGCG on the dephosphorylation of Rb protein :

### [Step 1] Cell line and cell culture

Cell lines used in this experiment and the culture thereof were the same as used in Step 1 of Example 1.

### [Step 2] Inhibition of UV-induced dephosphorylation of Rb protein by a combined treatment with ginsenoside F1 and EGCG

Cell lines cultured in Step 1 were treated with trypsin to give a single-cell suspension and seeded into 6-well microplates at 2×10⁵ cells per well, then cultured for 24 hours. Subsequently, the culture medium was refreshed with serum-free DMEM and cells were cultured for another 24 hours. The microplates were then treated with 2 µM ginsenoside F1; 10 µM EGCG; a combination of 2 µM ginsenoside F1 and 10 µM EGCG; 5 µM ginsenoside F1; and 50 µM EGCG, respectively. Ginsenoside F1 was dissolved in 100% ethanol at a 1/1000-fold concentration to the medium and EGCG (Sigma) was dissolved in DMSO at a 1/1000-fold concentration to the medium. After 24-hour treatment of each test sample, each microplate was washed with PBS and exposed to 60 mJ/cm² of UVB in the presence of PBS. PBS was then removed and the culture medium was refreshed with a medium containing each compound at the corresponding concentration. As a control, untreated cells were cultured in the same way.

24 hours after UV irradiation, cells treated with each compound or untreated were washed with PBS and harvested by treating with trypsin. Cells were then lysed in 500µℓ of protein extraction buffer containing 8M urea, 2% CHAPS, 50mM DTT, 2M thiourea, 2mM PMSF and 100µg/µℓ leupeptine at room temperature for 10 minutes. Supernatants were collected by centrifugation at 15,000xg for 10 minutes at 4°C and protein concentrations were determined using BIO-Rad Protein Dye Reagent^{™}. 20µg of proteins were separated in size order by 8% SDS-PAGE and transferred to PDF membrane (BioRad) at 50 V for 12 hours. The blots were blocked in a 5% non-fat milk-solution for 1 hour and then reacted with an anti-Rb rabbit polyclonal antibody (recognizing all forms of Rb (hyperphosphorylated form, underphosphorylated form, etc.); Santa Cruz) as a primary antibody and an HRP-conjugated anti-rabbit IgG (Amersham) as a secondary antibody using an ECL kit (Amersham). The blots were then exposed to X-ray film (Fuji) and developed to determine the level of protein expression. The same blots were washed twice, each time for 10 minutes, with a buffer containing 6.25mM Tris, 2% SDS and 100mM β-mercaptoethanol at 50°C. The blots were again blocked in a 5% non-fat milk-solution for 1 hour and then reacted with a monoclonal antibody anti-underphosphorylaed Rb (recognizing only underphosphorylated form of Rb; BD Biosciences) as a primary antibody and an HRP-conjugated anti-mouse IgG (Amersham) as a secondary antibody using an ECL kit (Amersham). The blots were then exposed to X-ray film (Fuji) and developed to determine the level of protein expression. The bands on the film were scanned using PowerLook 2100 XL (UMAX) and analyzed with ImageMaster 2D Elite software (Amersham Biosciences). The results are shown in Figure 5.

As shown in Figure 5, Rb protein was dephosphorylated by UV irradiation in cells treated with either of the two compounds alone or in untreated cells to be an underphosphorylated form. However, combined treatment with two compounds inhibited the dephosphorylation of Rb protein to produce all forms of Rb proteins.

Hereinafter, the external compositions containing ginsenoside F1 and EGCG according to the present invention were provided as the following formulations. However, the formulation of the present external compositions may not be limited hereto.

**[Table 1]**

| Formulation 1 : Skin softener | |
|---|---|
| Materials | Contents (wt%) |
| Betain | 4.0 |
| Natogum | 0.1 |
| Cellulose gum | 5.0 |
| Ethanol | 5.0 |
| Butylene glycol | 5.0 |
| Polyoxyethylene hydrogenated castor oil | 0.2 |
| Tocopheryl acetate | 5.0 |
| Preservative | q.s. |
| Pigments | q.s. |
| EGCG | 0.0005 |
| Ginsenoside F1 | 0.0001 |
| Distilled water | To 100 |

**[Table 2]**

| Formulation 2 : Nutrient toilet water | |
|---|---|
| Materials | Contents (wt%) |
| Cetyl ethyl hexanoate | 5.0 |
| Cetostearyl alcohol | 1.5 |
| Lipophilic monostearic stearate | 1.2 |
| Squalane | 2.0 |
| Polysorbate 60 | 1.2 |
| Sorbitan sesquioleate | 0.8 |
| Glycerin | 8.0 |
| Triethanolamine | 0.2 |
| Carboxyvinyl polymer | 0.2 |
| EGCG | 0.001 |
| Ginsenoside F1 | 0.001 |
| Preservative | q.s. |
| Pigments | q.s. |
| Perfume | q.s. |
| Distilled water | To 100 |

**[Table 3]**

| Formulation 3 : Nutrient cream | |
|---|---|
| Materials | Contents (wt%) |
| Beeswax | 1.0 |
| Glyceryl stearate | 2.0 |
| Cetostearate | 2.5 |
| Polysorbate 60 | 1.2 |
| Sorbitan sesquioleate | 0.4 |
| Cetyl ethyl hexanoate | 5.0 |
| Squalane | 7.0 |
| Liquid paraffin | 8.0 |
| Glycerin | 8.0 |
| Propylene glycol | 5.0 |
| Plant extracts | 5.0 |
| EGCG | 0.05 |
| Ginsenoside F1 | 0.01 |
| Preservative | q.s. |
| Pigments | q.s. |
| Perfume | q.s. |
| Distilled water | To 100 |

**[Table 4]**

| Formulation 4 : Essence | |
|---|---|
| Materials | Contents (wt%) |
| Glycerin | 15.0 |
| Propylene glycol | 5.0 |
| Cellulose gum | 0.1 |
| Natogum | 5.0 |
| Hyaluronic acid | 8.0 |
| Aminopropane sulfonic acid | 2.0 |
| Carboxyvinyl polymer | 0.3 |
| Ethanol | 5.0 |
| Polyoxyethylene hydrogenated castor oil | 0.5 |
| Triethanolamine | 0.3 |
| EGCG | 0.1 |
| Ginsenoside F1 | 0.01 |
| Preservative | q.s. |
| Perfume | q.s. |
| Pigments | q.s. |
| Distilled water | To 100 |

**[Table 5]**

| Formulation 5 : Lotion | |
|---|---|
| Materials | Contents (wt%) |
| Glyceryl stearate | 1.5 |
| Polysorbate 60 | 1.5 |
| Sorbitan sesquioleate | 0.5 |
| Cetyl ethyl hexanoate | 2.0 |
| Squalane | 3.0 |
| Glycerin | 8.0 |
| Carboxyvinyl polymer | 0.5 |
| Collagen hydrolysate | 1.0 |
| Triethanolamine | 0.5 |
| EGCG | 1.0 |
| Ginsenoside F1 | 0.5 |
| Preservative | q.s. |
| Perfume | q.s. |
| Pigments | q.s. |
| Distilled water | To 100 |

**[Table 6]**

| Formulation 6 : Ointment | |
|---|---|
| Materials | Contents (wt%) |
| Capric/caprylic triglyceride | 10.0 |
| Liquid paraffin | 10.0 |
| Sorbitan sesquioleate | 6.0 |
| Octyl Dodeth-25 | 10.0 |
| Cetyl ethyl hexanoate | 10.0 |
| Squalane | 1.0 |
| Salicylic acid | 1.0 |
| Propylene glycol | 15.0 |
| Sorbitol | 1.0 |
| EGCG | 0.01 |
| Ginsenoside F1 | 0.01 |
| Distilled water | To 100 |

**[Table 7]**

| Formulation 7 : Spray | |
|---|---|
| Materials | Contents (wt%) |
| Triethanolamine | 0.2 |
| Polyvinylpyrrolidone/vinyl acetate | 3.0 |
| Propylene glycol | 8.0 |
| Polyacrylate | 0.2 |
| EGCG | 0.0005 |
| Ginsenoside F1 | 0.0001 |
| Distilled water | To 100 |

### INDUSTRIAL APPLICATION OF THE INVENTION

As above described, a combined treatment with ginsenoside F1 and EGCG in the present invention can inhibit UV-induced apoptotic cell death by inhibiting UV-caused down-regulations of Bcl-2 expression or Bcl-2 transcription factor, Brn-3a expression by the synergistic interaction thereof even at low concentrations, where any single treatment shows no effect, and by preventing the dephosphorylation of Rb protein. Thus, the present invention provides the possibility of using a combination of ginsenoside F1 and EGCG as an anti-aging agent in human skin by preventing UV-caused cellular damages. Further, the present invention can supply high functional cosmetic products at low prices by using two expensive compounds at low concentrations (in the case of single treatments, 2.5-fold and 5-fold concentrations of ginsenoside F1 and EGCG respectively are necessary for the effects targeted).

## Claims

1. A skin-care composition containing 20-O-β-D-glucopyranosyl-20(S)-protopanaxatriol (ginsenoside F1) and (-)epigallocatechin-3-gallate (EGCG) as active ingredients.

2. The composition according to Claim 1, wherein said skin-care is obtained by the apoptosis-inhibitory effect of said active ingredients.

3. The composition according to Claim 2, wherein said apoptosis is apoptotic cell death induced by low dose of UV irradiation.

4. The composition according to Claims 2 or 3, wherein said apoptosis-inhibitory effect is obtained by regulating the expression of Bcl-2.

5. The composition according to Claim 4, wherein said regulation of Bcl-2 expression is obtained by regulating the expression of Brn-3a.

6. The composition according to any one of Claims 1 to 5, wherein said ginsenoside F1 and EGCG are incorporated in a combined amount of 0.0001% to 10% by weight based on the total weight of the composition.

7. The composition according to any one of Claims 1 to 5, wherein said ginsenoside F1 and EGCG are incorporated in a weight ratio of 1:0.1~10.

8. An inhibitor of Rb protein dephosphorylation containing ginsenoside F1 and EGCG as active ingredients.

9. An inhibitor of skin damage for preventing cellular damage caused by exposure to ultraviolet rays, containing a combination of low concentrations of ginsenoside F1 and EGCG as an active ingredient, wherein said ginsenoside F1 and EGCG are incorporated in a combined amount of 0.0001% to 10% by weight based on the total weight of the inhibitor.

## Patentansprüche

1. Hautpflegezusammensetzung enthaltend 20-O-β-D-Glucopyranosyl-20(S)-protopanaxatriol (Ginsenosid F1) und (-)Epigallocatechin-3-gallat (EGCG) als Wirkstoffe.

2. Zusammensetzung nach Anspruch 1, wobei die Hautpflege durch die Apoptose-hemmende Wirkung der Wirkstoffe erhalten wird.

3. Zusammensetzung nach Anspruch 2, wobei es sich bei der Apoptose um durch eine niedrige Dosis von UV-Strahlung induzierten apoptotischen Zelltod handelt.

4. Zusammensetzung nach den Ansprüchen 2 oder 3, wobei die Apoptose-hemmende Wirkung durch Regulieren der Expression von Bcl-2 erhalten wird.

5. Zusammensetzung nach Anspruch 4, wobei die Regulierung der Bcl-2-Expression durch Regulieren der Expression von Brn-3a erhalten wird.

6. Zusammensetzung nach einem der Ansprüche 1 bis 5, wobei das Ginsenosid F1 und EGCG in einer kombinierten Menge von 0,0001 bis 10 Gew.-%, bezogen auf das Gesamtgewicht der Zusammensetzung, eingearbeitet sind.

7. Zusammensetzung nach einem der Ansprüche 1 bis 5, wobei das Ginsenosid F1 und EGCG in einem Gewichtsverhältnis von 1:0,1 ~10 eingearbeitet sind.

8. Hemmer der Rb-Protein-Dephosphorylierung enthaltend Ginsenosid F1 und EGCG als Wirkstoffe.

9. Hemmer einer Hautschädigung zum Verhüten einer zellulären Schädigung, die durch Einwirkung von Ultraviolettstrahlen verursacht wird, enthaltend eine Kombination von niedrigen Konzentrationen von Ginsenosid F1 und EGCG als Wirkstoff, wobei das Ginsenosid F1 und EGCG in einer kombinierten Menge von 0,0001 bis 10 Gew.-%, bezogen auf das Gesamtgewicht des Hemmers, eingearbeitet sind.

## Revendications

1. Une composition de soin de la peau contenant du 20-O-ß-D-glßucopyranosyl-20(S)protopanaxatriol (ginsénoside F1) et du (-)epigallocatéchin-3-gallate (EGCG) en tant qu'ingrédients actifs.

2. La composition selon la revendication 1, dans laquelle ledit soin de la peau est obtenu par l'effet inhibiteur de l'apoptose desdits ingrédients actifs.

3. La composition selon la revendication 2, dans laquelle ladite apoptose est une mort cellulaire apoptotique induite par une faible dose de rayonnement UV.

4. La composition selon la revendication 2 ou 3, dans laquelle ledit effet inhibiteur de l'apoptose est obtenu en régulant l'expression de Bcl-2.

5. La composition selon la revendication 4, dans laquelle ladite régulation de l'expression de Bcl-2 est obtenue en régulant l'expression de Brn-3a.

6. La composition selon l'une quelconque des revendications 1 à 5, dans laquelle lesdits ginsénoside F1 et EGCG sont incorporés en une quantité combinée de 0,0001% à 10% en poids par rapport au poids total de la composition.

7. La composition selon l'une quelconque des revendications 1 à 5, dans laquelle lesdits ginsénoside F1 et EGCG sont incorporés dans un rapport pondéral de 1:0,1~10.

8. Un inhibiteur de la déphosphorylation de la protéine Rb contenant du ginsénoside F1 et du EGCG en tant qu'ingrédients actifs.

9. Un inhibiteur des dommages cutanés pour prévenir les dommages cellulaires causés par l'exposition aux rayons ultraviolets, contenant une combinaison de faibles concentrations de ginsénoside F1 et de EGCG en tant qu'ingrédient actif, dans lequel lesdits ginsénoside F1 et EGCG sont incorporés en une quantité combinée de 0,0001% à 10% en poids par rapport au poids total de l'inhibiteur.
